(19) Europäisches Patentamt
European Patent Office
Office européen des brevets

(11) Veröffentlichungsnummer: **0 027 177**
**B1**

(12) # EUROPÄISCHE PATENTSCHRIFT

(45) Veröffentlichungstag der Patentschrift: **02.05.84**

(21) Anmeldenummer: **80105338.0**

(22) Anmeldetag: **06.09.80**

(51) Int. Cl.³: **C 07 D 233/60,**
C 07 D 249/08 //A01N43/50,
A01N43/64, C07C49/16

(54) **1-Azolyl-1-halogen-alkan-2-one und Verfahren zur Herstellung von 1-Azolyl-1-phenoxy-alkan-2-onen.**

(30) Priorität: **18.09.79 DE 2937595**

(43) Veröffentlichungstag der Anmeldung:
**22.04.81 Patentblatt 81/16**

(45) Bekanntmachung des Hinweises auf die Patenterteilung:
**02.05.84 Patentblatt 84/18**

(84) Benannte Vertragsstaaten:
**BE CH DE FR GB IT LI NL**

(56) Entgegenhaltungen:
**EP - A - 0 000 017**
**EP - A - 0 005 142**
**EP - A - 0 026 990**
**DE - A - 2 406 665**
**US - A - 4 251 540**

**Houben-Weyl, Band 6/3, Seite 55-56**
**Houben-Weyl, Band XI/1, Seite 24ff und Band XI/2, Seite 591ff**
**H. Böhme, Iminium Salts in Organic Chemistry, part 1, Seite 162**

(73) Patentinhaber: **BAYER AG**
**Konzernverwaltung RP Patentabteilung**
**D-5090 Leverkusen 1 Bayerwerk (DE)**

(72) Erfinder: **Holmwood, Graham, Dr.**
**Katernbergerstrasse 184**
**D-5600 Wuppertal 1 (DE)**
Erfinder: **Oeckl, Siegfried, Dr.**
**Andreas Gryphius Strasse 9**
**D-5000 Köln 80 (DE)**
Erfinder: **Stetter, Jörg, Dr.**
**Gellertweg 4**
**D-5600 Wuppertal 1 (DE)**

**EP 0 027 177 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

1-Azolyl-1-halogen-alkan-2-one und Verfahren zur Herstellung von 1-Azolyl-1-phenoxy-alkan-2-onen

Die vorliegende Erfindung betrifft eine neues Verfahren zur Herstellung von bekannten, fungizid wirksamen, 1-Azolyl-1-phenoxy-alkan-2-onen.

Es ist bereits bekannt geworden, daß man 1-Azolyl-1-phenoxy-alkan-2-one, wie insbesondere 1-Azolyl-3,3-dimethyl-1-phenoxy-butan-2-one, und 1-Azolyl-1-phenoxy-2-phenyl-ethan-2-one erhält, wenn man 3,3-Dimethyl-1-phenoxy-butan-2-one bzw. 1-Phenoxy-2-phenyl-ethan-2-one in üblicher Weise halogeniert und die dabei entstehenden 1-Chlor(Brom)-3,3-dimethyl-1-phenoxy-butan-2-one bzw. 1-Chlor(Brom)-1-phenoxy-2-phenyl-ethan-2-one mit Triazol oder Imidazol in Gegenwart von polaren organischen Lösungsmitteln, wie insbesondere Ketone, und in Gegenwart eines Säureakzeptors bei Temperaturen zwischen 20 und 120°C umsetzt (vergleiche z.B. die Deutschen Offenlegungsschriften 21 05 490, 22 01 063, 24 01 715, 27 05 678, 27 05 676 und 27 13 777.

Dieses Verfahren hat den Nachteil, daß bei den üblichen Halogenierungen von insbesondere im Phenylteil empfindlichen Phenolethern Nebenreaktionen auftreten können, wodurch das Verfahren in seiner Gesamtheit unwirtschaftlich werden kann.

Weiterhin ist es bekannt geworden, daß die 3,3-Dimethyl-1-phenoxy-1-(1,2,4-triazol-1-yl)-butan-2-one auch erhalten werden, wenn man Dihalogenpinakolin mit 1,2,4-Triazol und mit Phenolen in Gegenwart eines Säurebindemittels und eines Verdünnungsmittels bei Temperaturen zwischen 0 und 150°C umsetzt (vergleiche Deutsche Offenlegungsschrift 2 406 665.

Dieses Verfahren hat den Nachteil, daß Konkurrenzreaktionen zur verstärken Bildung von Nebenprodukten führen, die die Ausbeute beträchtlich verringern und eine umständliche sowie zeitraubende Aufarbeitung erforderlich machen.

Es wurde gefunden, daß man die bekannten 1-Azolyl-1-phenoxy-alkan-2-one der Formel

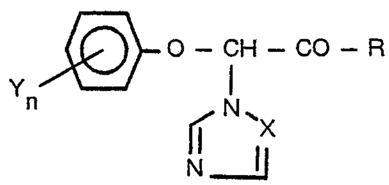

(I)

in welcher

R für Alkyl mit 1 bis 4 Kohlenstoffatomen oder für gegebenenfalls durch Halogen, Alkyl mit 1 bis 4 oder Alkoxy mit 1 bis 2 Kohlenstoffatomen substituiertes Phenyl steht,

X für ein Stickstoffatom oder die CH—Gruppe steht,

Y für Halogen, Alkyl und Alkoxy mit jeweils 1 bis 4 und Cycloalkyl mit 5 bis 7 Kohlenstoffatomen steht, für die Nitro-Gruppe und für die Gruppierung —CO—OR' steht, ferner für Phenyl und Phenoxy steht, wobei die beiden letztgenannten Reste substituiert sein können durch Halogen oder Alkyl mit 1 bis 2 Kohlenstoffatomen,

R' für Alkyl mit 1 bis 4 Kohlenstoffatomen und für Phenyl oder Benzyl steht, wobei die beiden letztgenannten Reste durch Halogen und Alkyl mit 1 bis 2 Kohlenstoffatomen substituiert sein können und

n für die Zahlen 0, 1, 2 oder 3 steht,

erhält, wenn man Azolylhalogenketone der Formel

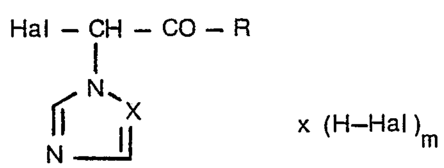

(II)

in welcher

R und X die oben angegebene Bedeutung haben,

Hal für Fluor, Chlor oder Brom steht und

m für 0 oder 1 steht,

mit Phenolen der Formel

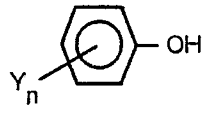

(III)

in welcher

Y und n die oben angegebene Bedeutung haben,

in gegenwart eines inerten organischen Lösungsmittel und in Gegenwart eines anorganischen oder organischen Säurebindemittels im Temperaturbereich zwischen 0 und 140°C umsetzt.

Es ist als überraschend zu bezeichnen, daß das erfindungsgemäße Verfahren breit anwendbar ist und es hat somit den Vorteil, unabhängig von der Empfindlichkeit der Phenole gute Ausbeuten zu liefern. Die guten Ausbeuten sind auch insofern überraschend, als daß die Azolylhalogenketone der Formel (II), insbesondere die Chlor-und Brom-Derivate, als freie Basen eine ausgeprägte Neigung zur Selbstquarternisierung aufweisen.

Die erfindungsgemäß herstellbaren 1-Azolyl-1-phenoxy-alkan-2-one sind durch die Formel (I) allgemein definiert.

Bevorzugt sind diejenigen Verbindungen der Formel (I), in denen *R* für tert.-Butyl, Isopropyl, Phenyl, Chlorphenyl, Fluorphenyl oder Dichlorphenyl steht; *X* für ein Stickstoffatom oder die CH—Gruppe steht; *Y* für Fluor, Chlor, Brom, Methyl, Ethyl, Methoxy, Methoxycarbonyl, Cyclohexyl, Phenyl, Chlorphenyl, Phenoxy, Chlorphenoxy oder Nitro steht.

Verwendet man beispielsweise 1-Brom-3,3-dimethyl-1-(1,2,4-triazol-1-yl)-butan-2-on und 4-Chlorphenol als Ausgangsstoffe, so kann der Reaktionsablauf durch das folgende Formelschema wiedergegeben werden:

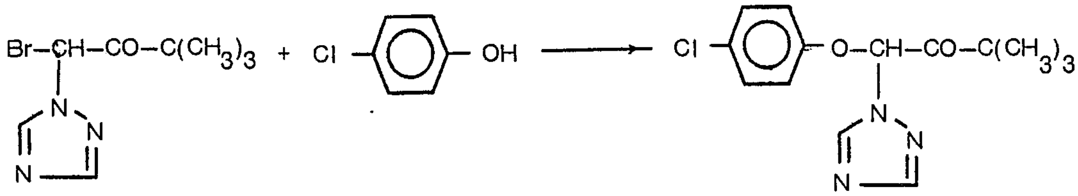

Die bei der Durchführung des erfindungsgemäßen Verfahrens als Ausgangsstoffe benötigten Azolylhalogenketone sind durch die Formel (II) allgemein definiert. In dieser Formel stehen *R* und *X* vorzugsweise für diejenigen Reste, die bereits im Zusammenhang mit der Beschreibung der erfindungsgemäß herstellbaren Stoffe der Formel (I) vorzugsweise für diese Reste genannt wurden. *Hal* steht vorzugsweise für Fluor, Chlor oder Brom und der Index *m* steht für 0 oder 1.

Die Azolylhalogenketone der Formel (II) sind teilweise bekannt (vgl. DE—OS 27 56 269). Noch nicht bekannt sind die Azolylhalogenketone der Formel

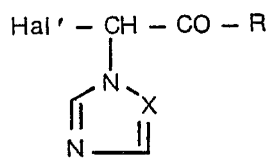 $x\ (H{-}Hal')_m$ (IIa)

in welcher

X für ein Stickstoffatom oder die CH—Gruppe steht,

R für Alkyl oder gegebenenfalls substituiertes Phenyl steht,

Hal' für Fluor steht, sowie zusätzlich auch für Chlor und Brom steht, wenn *R* für gegebenenfalls substituiertes Phenyl steht und

m für 0 oder 1 steht.

In den neuen Verbindungen der Formel (IIa) steht *X* für ein Stickstoffatom oder die CH—Gruppe. *R* steht für geradkettiges oder verzweigtes Alkyl mit 1 bis 4 Kohlenstoffatomen sowie für gegebenenfalls substituiertes Phenyl, wobei als Substituenten folgende in Frage kommen: Halogen, Alkyl mit 1 bis 4 und Alkoxy mit 1 bis 2 Kohlenstoffatomen. *Hal'* steht für Fluor sowie zusätzlich auch für Chlor und Brom, wenn *R* für gegebenenfalls substituiertes Phenyl steht. Der Index m steht für 0 oder 1.

Bevorzugt sind diejenigen Verbindungen der Formel (IIa), in denen *X* für ein Stickstoffatom oder die CH—Gruppe steht; *R* für tert.-Butyl, Isopropyl, Phenyl, Chlorphenyl, Fluorphenyl oder Dichlorphenyl steht; *Hal'* für Fluor steht, sowie zusätzlich auch für Chlor und Brom steht, wenn *R* für gegebenenfalls substituiertes Phenyl steht; und der Index *m* für 0 oder 1 steht.

Die Azolylhalogenketone der Formel (II) werden erhalten, indem man

a) Azolylketone der Formel

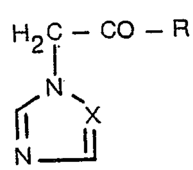 (IV)

in welcher

R und X die oben angegebene Bedeutung haben,

mit Brom oder Chlor in Gegenwart eines sauren Lösungsmittels und gegebenenfalls in Gegenwart eines Halogenwasserstoffakzeptors umsetzt und gegebenenfalls in den entsprechenden Azolyl-brom(chlor)-ketonen das Brom(Chlor) in üblicher Weise gegen Fluor austauscht (vergleiche auch die Herstellungsbeispiele).

Die Azolyl-fluorketone der Formel (II) können auch erhalten werden, indem man

b) Halogenketone der Formel

$$\underset{\displaystyle Z}{\overset{}{F-\underset{|}{C}H-CO-R}} \qquad (V)$$

in welcher

R die oben angegebene Bedeutung hat und

Z für Chlor oder Brom steht,

mit Imidazol oder 1,2,4-Triazol in Gegenwart eines Säurebindemittels und in Gegenwart eines Lösungsmittels umsetzt (vergleiche auch die Herstellungsbeispiele).

Die für die Variante (a) als Ausgangsstoffe zu verwendenden Azolylketone der Formel (IV) sind bekannt (vergleiche DE—OS 26 38 470 und DE—OS 24 31 407 und können erhalten werden, indem man bekannte Halogenide der Formel

$$Br(Cl)-CH_2-CO-R \qquad (VI)$$

in welcher

R die oben angegebene Bedeutung hat,

mit Imidazol oder 1,2,4-Triazol in Gegenwart eines Säurebindemittels, wie z.B. Kaliumcarbonat, und in Gegenwart eines inerten organischen Lösungsmittels, wie z.B. Aceton, bei Temperaturen zwischen 60 und 120°C umsetzt.

Die für die Variante (b) als Ausgangsstoffe zu verwendenden Halogenketone der Formel (V) sind noch nicht bekannt. Sie werden erhalten, indem man in den bekannten Halogeniden der Formel (VI) das Brom(Chlor) in üblicher Weise gegen Fluor austauscht und in den entsprechenden Fluorketonen der Formel

$$F-CH_2-CO-R \qquad (VII)$$

in welcher

R die oben angegebene Bedeutung hat,

eines der aktiven Wasserstoffatome gemäß Variante (a) gegen Chlor oder Brom austauscht (vergleiche auch die Herstellungsbeispiele).

Bei der Herstellung der Ausgangsstoffe der Formel (II) gemäß Verfahrensvariante (a) kommen als Lösungsmittel vorzugsweise sauer Lösungsmittel infrage, wie z.B. Essigsäure oder wässrige Bromwasserstoffsäure.

Als Halogenwasserstoffakzeptoren können bei der Durchführung der Verfahrensvariante (a) alle üblicherweise verwendbaren organischen und insbesondere anorganischen Basen eingesetzt werden, wie beispielsweise Natrium- und Kalium-hydroxid, -carbonat oder -acetat.

Die Reaktionstemperaturen können bei der Durchführung der Verfahrensvariante (a) in einem größeren Bereich variiert werden. Im allgemeinen arbeitet man bei Temperaturen zwischen 0 bis 120°C, vorzugsweise bei 20 bis 100°C.

Bei der Durchführung der Verfahrensvariante (a) arbeitet man vorzugsweise in molaren Mengen. Die Isolierung der Verbindungen der Formel (II) erfolgt in Form der freien Base oder als Halogensalz, jeweils in allgemein üblicher Art und Weise.

Der eventuelle Halogenaustausch gegen Fluor erfolgt in üblicher Weise.

Bei der Herstellung der Ausgangsstoffe der Formel (II) gemäß Verfahrensvariante (b) kommen als Lösungsmittel vorzugsweise inerte organische Lösungsmittel infrage. Hierzu gehören vorzugsweise Ketone, wie insbesondere Aceton; Nitrile, wie Propionitril, insbesondere Acetonitril; Alkohole, wie Ethanol oder Isopropanol; Ether, wie Tetrahydrofuran oder Dioxan; aromatische Kohlenwasserstoffe, wie Benzol oder Toluol; und Formamide, wie insbesondere Dimethylformamid.

Die Umsetzung gemäß Verfahrensvariante (b) wird in Gegenwart eines Säurebinders vorgenommen. Man kann alle üblicherweise verwendbaren anorganischen und organischen Säurebinder zugeben, wie Alkalicarbonate, beispielsweise Natriumcarbonat, Kaliumcarbonat und Natriumhydrogencarbonat, oder wie niedere tertiäre Alkylamine, Cycloalkylamine oder Aralkylamine, beispielsweise Triethylamin, N,N-Dimethylcyclohexylamin, N,N-Dimethylbenzylamin. Vorzugsweise verwendet man einen entsprechenden Ueberschuß an Imidazol oder 1,2,4-Triazol.

Die Reaktionstemperaturen können bei der Durchführung der Verfahrensvariante (b) in einem

größeren Bereich variiert werden. Im allgemeinen arbeitet man zwischen 20 und 150°C, vorzugsweise bei 60 bis 120°C. Zweckmäßigerweise kann vorzugsweise bei der Siedetemperatur des Lösungsmittels gearbeitet werden.

Bei der Durchführung der Verfahrensvariante (b) setzt man vorzugsweise auf 1 Mol der Verbindungen der Formel (V) 2 Mol Imidazol bzw. 1,2,4-Triazol und 1 bis 2 Mol Säurebinder ein. Die Isolierung der Verbindungen der Formel (II) erfolgt in allgemein überlicher Art und Weise.

Als Ausgangsstoffe der Formel (II) seien außer den bei den Herstellungsbeispielen genannten Verbindungen die folgenden genannt:

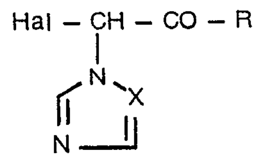 x $(H{-}Hal)'_m$ (II)

| Hal | X | R | m |
|---|---|---|---|
| Br | CH | $C(CH_3)_3$ | 1 |
| Cl | CH | $C(CH_3)_3$ | 1 |
| Br | N | $C(CH_3)_3$ | 0 |
| Cl | N | $C(CH_3)_3$ | 0 |
| Br | CH | 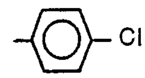 | 1 |
| Cl | CH | 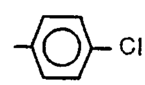 | 1 |
| Br | N | 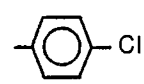 | 0 |
| Cl | N | 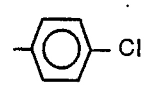 | 0 |
| F | CH | $C(CH_3)_3$ | 0 |
| F | N | $C(CH_3)_3$ | 0 |

Für die erfindungsgemäße Umsetzung kommen als Verdünnungsmittel vorzugsweise inerte organische Lösungsmittel infrage. Hierzu gehören vorzugsweise Ether, wie Diethylether, Alkohole, wie Methanol; Ketone, wie Aceton; aromatische Kohlenwasserstoffe, wie Benzol; sowie auch Dimethylformamid und Dimethylsulfoxid.

Die erfindungsgemäße Umsetzung wird in Gegenwart eines Säurebinders vorgenommen. Man kann alle üblicherweise verwendbaren anorganischen oder organischen Säurebinder zugeben, wie Alkalimetallcarbonate, beispielsweise Kaliumcarbonat oder Natriumcarbonat, Alkalimetallhydroxide, beispielsweise Natriumhydroxid, Alkylimetallalkoholate, beispielsweise Natriummethylat, oder wie niedere tertiäre Alkylamine, beispielsweise Triethylamin.

Die Reaktiontemperaturen können bei der Durchführung des erfindungsgemäßen Verfahrens in einem größeren Bereich variiert werden. Man arbeitet zwischen 0 und 140°, vorzugsweise zwischen 50 und 100°C.

Bei der Durchführung des erfindungsgemäßen Verfahrens setzt man auf 1 Mol der Verbindung der Formel (II) vorzugsweise 1 bis 4 Mol Phenol der Formel (III) ein. Die Isolierung der Verbindungen der Formel (I) erfolgt in üblicher Weise. Die Verbindungen der Formel (II) können vorzugsweise in form ihrer Hydrohalogenide eingesetzt werden.

Die erfindungsgemäß herstellbaren Wirkstoffe zeichnen sich bekanntermaßen durch sehr gute fungizide Wirksamkeit aus (vgl. DE—AS 22 01 063, DE—OS 23 25 156, DE—OS 27 05 676 und DE—OS 27 05 678). So können sie beispielsweise mit besonders gutem Erfolg als Mittel gegen echten Mehltau (als Blattfungizid) und gegen Erkrankungen des Getreides, wie Getreiderost (als Saatgutbeizmittel) verwendet werden.

*Herstellungsbeispiele*

Beispiel 1

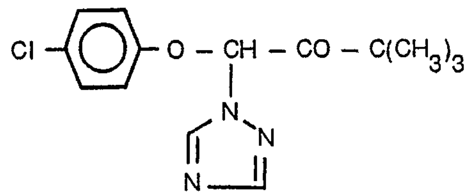

34,8 g (0,27 Mol) 4-Chlorphenol werden in 250 ml absolutem Methanol gelöst und mit 6,22 g (0,27 Mol) Natrium versetzt. Nach vollständiger Auflösung des Natriums wird die Lösung 30 Minuten unter Rückfluß erhitzt, dann werden bei leichtem Sieden 22,2 g (0,09 Mol) 1-Brom-3,3-dimethyl-1-(1,2,4-triazol-1-yl)-butan-2-on in 30 ml absolutem Methanol zugetropft. Die Reaktionsmischung wird über Nacht unter Rückfluß erhitzt und nach dem Abkühlen eingeengt. Der Rückstand wird zwischen Ether und Wasser verteilt, die organische Phase abgetrennt, dreimal mit 1 n Natronlauge, zweimal mit Wasser und einmal mit Natriumchloridlösung gewaschen und über Natriumsulfat getrocknet. Nach Abdestillieren des Lösungsmittels kristallisiert der Rückstand nach Zugabe von n-Hexan. Man erhält 15,2 g (57,5% der Theorie) 1-(4-Chlorphenoxy)-3,3-dimethyl-1-(1,2,4-triazol-1-yl)-butan-2-on vom Schmelzpunkt 74—76°C.

Beispiel 2

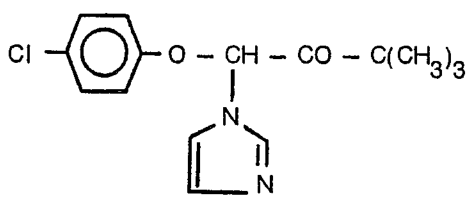

23,14 g (0,18 Mol) 4-Chlorphenol werden in 200 ml absolutem Methanol gelöst und mit 4,14 g (0,18 Mol) Natrium versetzt. Nach vollständiger Auflösung des Natriums wird die Lösung 30 Minuten unter Rückfluß erhitzt, dann werden bei leichtem Sieden 19,56 g (0,06 Mol) 1-Brom-3,3-dimethyl-1-(imidazol-1-yl)-butan-2-on-hydrobromid zugegeben. Die Reaktionsmischung wird über Nacht unter Rückfluß erhitzt und nach dem Abkühlen eingeengt. Der Rückstand wird zwische Ether und Wasser verteilt, die organische Phase abgetrennt, dreimal mit 1 n Natronlauge, zweimal mit Wasser und dreimal mit Natriumchloridlösung gewaschen und über Natriumsulfat getrocknet. Nach Abdestillieren des Lösungsmittels wird der Rückstand aus Cyclohexan umkristallisiert. Man erhält 13,9 g (70% der Theorie) 1-(4-Chlorphenoxy)-3,3-dimethyl-1-(imidazol-1-yl)-butan-2-on vom Schmelzpunkt 95—97°C.

Beispiel 3

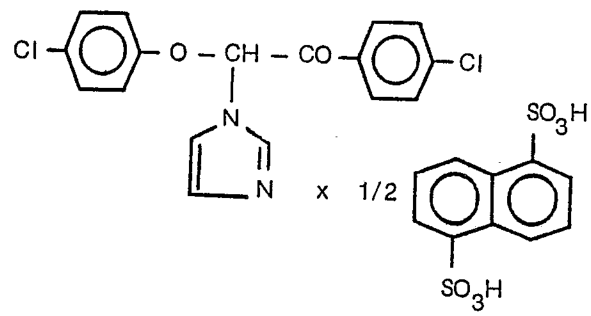

23,14 g (0,18 Mol) 4-Chlorphenol werden in 200 ml absolutem Methanol gelöst und mit 4,14 g (0,18 Mol) Natrium versetzt. Nach vollständiger Auflösung des Natriums wird die Lösung 30 Minuten unter Rückfluß erhitzt, dann werden bei leichtem Sieden 22,83 g (0,06 Mol) ω-Brom-ω-(imidazol-1-yl)-4-chloracetophenon-hydrobromid zugegeben. Die Reaktionsmischung wird über Nacht unter Rückfluß erhitzt und nach dem Abkühlen eingeengt. Der Rückstand wird zwischen Ether und Wasser verteilt, die organische Phase abgetrennt, dreimal mit 1 n Natronlauge, zweimal mit Wasser und einmal mit Natriumchloridlösung gewaschen und über Natriumsulfat getrocknet. Nach Abdestillieren des Lösungsmittels wird der Rückstand in Ether aufgenommen und mit 1 n Salzsäure extrahiert. Die saure wässrige Phase wird auf festes Natriumhydrogencarbonat gegeben, mit Ether extrahiert, über Natriumsulfat getrocknet und durch Abdestillieren des Lösungsmittels eingeengt. Der Rückstand wird aus n-Hexan/Ether = 8/2 umkristallisiert und wieder in Ether aufgenommen und eingeengt. Der ölige Rückstand (16,8 g) wird mit 8,45 g 1,5-Naphthalindisulfonsäure umgesetzt. Man erhält 17,5 g (59% der Theorie) 1 - (4 - Chlorphenoxy) - 2 - (4 - chlorphenyl) - 1 - (imidazol - 1 - yl) - ethan - 2 - on - naphthalindisulfonat - (1,5) vom Schmelzpunkt 290—292°C.

In analoger Weise werden die in der folgenden Tabelle 1 formelmäßig aufgeführten Verbindungen erhalten:

TABELLE 1

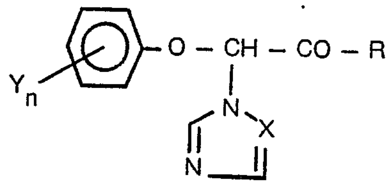

(I)

| Bsp. Nr. | $Y_n$ | X | R | Schmelzpunkt (°C) oder Siedepunkt (°C)/ mm Hg-Säule |
|---|---|---|---|---|
| 4 | 4–F | N | $C(CH_3)_3$ | 160/0,3 |
| 5 | 4–Br | N | $C(CH_3)_3$ | 89–92 |
| 6 | 4–$NO_2$ | N | $C(CH_3)_3$ | 145 |
| 7 | 2,4–$Cl_2$ | N | $C(CH_3)_3$ | 65 |
| 8 | 3–Cl | N | $C(CH_3)_3$ | 65–67 |
| 9 | 4–Br, 2–Cl | N | $C(CH_3)_3$ | 94–96 |
| 10 | 2–$OCH_3$ | N | $C(CH_3)_3$ | 87 |
| 11 | 2,4–$(CH_3)_2$ | N | $C(CH_3)_3$ | 74 |
| 12 | 3,4–$Cl_2$ | N | $C(CH_3)_3$ | 82–84 |
| 13 | 3–Cl, 4–$NO_2$ | N | $C(CH_3)_3$ | 100–104 |
| 14 | 2–$CH_3$, 5–$NO_2$ | N | $C(CH_3)_3$ | 154 |
| 15 | 2–Br, 4 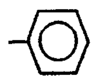 | N | $C(CH_3)_3$ | 125 |
| 16 | 4 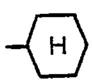 | N | $C(CH_3)_3$ | 98–99 |
| 17 | 2 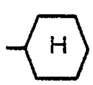 | N | $C(CH_3)_3$ | 107 |
| 18 | 4–O 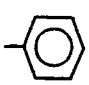 | N | $C(CH_3)_3$ | 98 |
| 19 | 2,6–$Cl_2$, 4 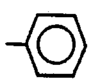 | N | $C(CH_3)_3$ | 149–50 |

TABELLE 1 (fortsetzung)

| Bsp. Nr. | $Y_n$ | X | R | Schmelzpunkt (°C) oder Siedepunkt (°C)/ mm Hg-Säule |
|---|---|---|---|---|
| 20 | 2,4,6–$Cl_3$ | N | $C(CH_3)_3$ | 150–160 (× HCl) |
| 21 | 2–Cl , 4 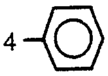 | N | $C(CH_3)_3$ | 107 |
| 22 | – | CH | $C(CH_3)_3$ | 147/0,01 |
| 23 | 3–Cl | CH | $C(CH_3)_3$ | 172 |
| 24 | 4–Br | CH | $C(CH_3)_3$ | 106 |
| 25 | 4–F | CH | $C(CH_3)_3$ | 166/0,01 |
| 26 | 4–$NO_2$ | CH | $C(CH_3)_3$ | 151 |
| 27 | 4–$C(CH_3)_3$ | CH | $C(CH_3)_3$ | 140 (× HCl) |
| 28 | 2 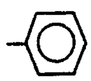 | CH | $C(CH_3)_3$ | 105 |
| 29 | 4 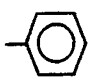 | CH | $C(CH_3)_3$ | 104 |
| 30 | 2,4–$Cl_2$ | CH | $C(CH_3)_3$ | 69 |
| 31 | 2,6–$Cl_2$ | CH | $C(CH_3)_3$ | 119 |
| 32 | 2,5–$Cl_2$ | CH | $C(CH_3)_3$ | 146 |
| 33 | 2–Cl , 6 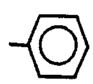 | CH | $C(CH_3)_3$ | 155 |
| 34 | 4–Br, 2–Cl | CH | $C(CH_3)_3$ | 123 (× HCl) |
| 35 | 2–$CH_3$, 5–$NO_2$ | CH | $C(CH_3)_3$ | 231 (× HCl) |
| 36 | 2–Cl , 4 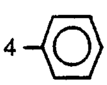 | CH | $C(CH_3)_3$ | 200–201 |
| 37 | 2,6–$Cl_2$, 4 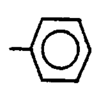 | CH | $C(CH_3)_3$ | 112–115 |
| 38 | 4–J | CH | $C(CH_3)_3$ | 90–91 |

TABELLE 1 (fortzetsung)

| Bsp. Nr. | $Y_n$ | X | R | Schmelzpunkt (°C) oder Siedepunkt (°C)/ mm Hg-Säule |
|---|---|---|---|---|
| 39 | 4–O–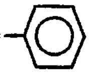 | CH | $C(CH_3)_3$ | 94–97 |
| 40 | 4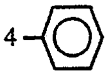 | N | $C(CH_3)_3$ | 105–06 |
| 41 | 4–Cl | N | 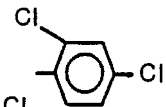 | 138–40 (x HCl) |
| 42 | 2,4–$Cl_2$ | N | 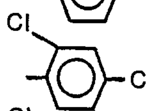 | 173–83 (x HCl) |
| 43 | 4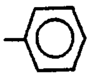 | N | 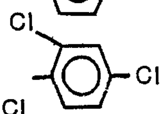 | 205–08 (x HCl) |
| 44 | 4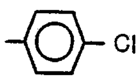 | N | 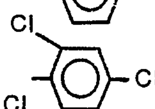 | 138 (Zers.) (x HCl) |
| 45 | 4–F | N | 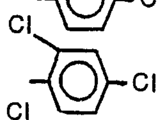 | 83–86 |
| 46 | 2,6–$Cl_2$ | N | 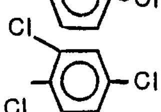 | 176 (x HCl) |
| 47 | 3–Cl | N | 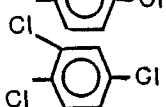 | 140–42 (x HCl) |
| 48 | – | N | 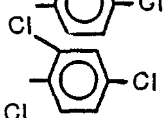 | 86–88 |
| 49 | 4–$CH_3$ | N | 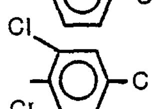 | 172 (x HCl) |
| 50 | 4–Cl, 2–$CH_3$ | N | 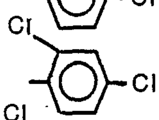 | 138 (x HCl) |
| 51 | 4–J | N | 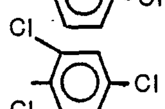 | 156 (x HCl) |
| 52 | 4–Cl | CH | 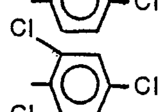 | 146–48 (x HCl) |
| 53 | 2,4–$Cl_2$ | CH | 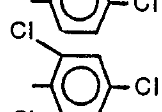 | 205–15 (x HCl) |
| 54 | 4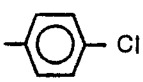 | CH | 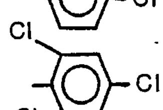 | 145–48 (x HCl) |
| 55 | 4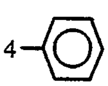 | CH | 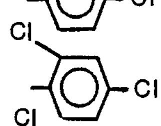 | 160–62 (x HCl) |
| 56 | 4–F | CH | 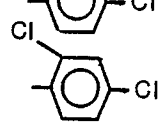 | 160 (x HCl) |

TABELLE 1 (fortzetsung)

| Bsp. Nr. | $Y_n$ | X | R | Schmelzpunkt (°C) oder Siedepunkt (°C)/ mm Hg-Säule |
|---|---|---|---|---|
| 57 | – | CH | 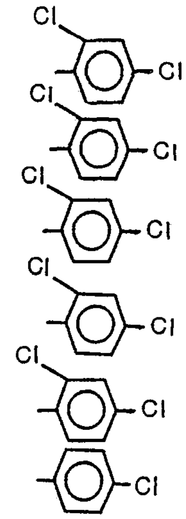 | 162–68 (x HCl) |
| 58 | 2,6–$Cl_2$ | CH | | 180 (x HCl) |
| 59 | 3–Cl | CH | | 168–71 (x HCl) |
| 60 | 4–CH | CH | | 110 (x HCl) |
| 61 | 4–Cl, 2–$CH_3$ | CH | | 177–78 (x HCl) |
| 62 | 2,4–$Cl_2$ | N | | 148–51 (x HCl) |
| 63 | 4–CO–$OCH_3$ | N | $C(CH_3)_3$ | 85–88 |

*Herstellung der Ausgangsstoffe der Formel (II)*

Beispiel (II—1)

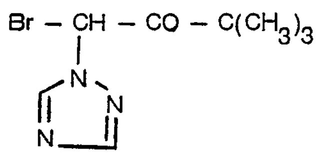

(Verfahren a)

Zu 83,5 g (0,5 Mol) 3,3-Dimethyl-1-(1,2,4-triazol-1-yl)-butan-2-on und 41 g (0,5 Mol) wasserfreiem Natriumacetat in 250 ml Eisessig werden unter Rühren bei 40 bis 50°C langsam 80 g (0.5 Mol) Brom zugetropft. Man läßt bei 40°C bis zur völligen Entfärbung weiterrühren. Danach gibt man die Reaktionsmischung auf 400 ml Wasser und extrahiert dreimal mit je 100 ml Chloroform. Die vereinigten organischen Phasen werden zunächst mit Natriumhydrogencarbonatlösung bis zur Beendigung der $CO_2$-Entwicklung und anschließend mit Wasser gewaschen, über Natriumsulfat getrocknet und im Vakuum durch Abdestillieren des Lösungsmittels eingeengt. Man erhält quantitativ rohes 1-Brom-3,3-dimethyl-1-(1,2,4-triazol-1-yl)-butan-2-on, das direkt weiter umgesetzt wird.

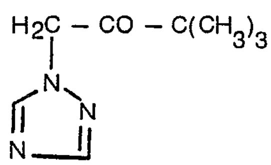

134.5 g (1 Mol) 1-Chlor-3,3-dimethyl-butan-2-on, 69 g (1 Mol) 1,2,4-Triazol und 140 g (1 Mol) gepulvertes Kaliumcarbonat werden in 500 ml Aceton unter Rühren 6 Stunden unter Rückfluß erhitzt. Danach läßt man abkühlen, filtriert das anorganische Salz ab und engt das Filtrat im Vakuum ein. Der ölige Rückstand kristallisiert nach dem Behandeln mit Diisopropylether aus. Man erhält nach dem Trocknen 123,6 g (74% der Theorie) 3,3-Dimethyl-1-(1,2,4-triazol-1-yl)-butan-2-on vom Schmelzpunkt 63—65°C.

Beispiel (II—2)

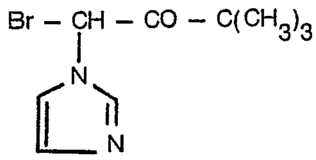

x HBr

(Verfahren a)

50 g (0,3 Mol) 3,3-Dimethyl-1-(imidazol-1-yl)-butan-2-on werden in 50 ml Bromwasserstoff (48%-ige wässrige Lösung) gelöst und bei 80 bis 95°C tropfenweise mit 47,9 g (0,3 Mol) Brom versetzt. Man läßt ca. 15 Minuten nachrühren und engt anschließend ein. Der hochviskose hellrote Niederschlag kristallisiert nach Animpfen langsam durch. Nach Umkristallisation aus Isopropanol erhält man 58,8 g (60% der Theorie) 1-Brom-3,3-dimethyl-1-(imidazol-1-yl)-butan-2-on-hydrobromid vom Schmelzpunkt 171—173°C.

Beispiel (II—3)

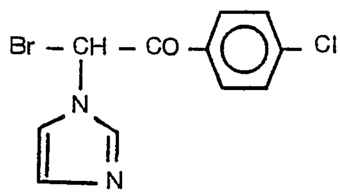

x HBr

(Verfahren a)

50 g (0,23 Mol) ω-(Imidazol-1-yl)-4-chloracetophenon werden in 80 ml Bromwasserstoff (40%-ige wässrige Lösung) vorgelegt und bei 100°C tropfenweise mit 36,21 g (0,23 Mol) Brom versetzt. Man läßt ca. 15 Minuten nachrühren. Das erstarrte Reaktionsgemisch wird mit 100 ml Bromwasserstoff versetzt, verrührt und abgesaugt. Der feste Rückstand wird in kaltem Ethanol verrührt, abgesaugt und getrocknet. Man erhält 74,4 g (85% der Theorie) ω-Brom-ω-(imidazol-1-yl)-4-chloracetophenon-hydrobomid vom Schmelzpunkt 228—231°C.

Beispiel (II—4)

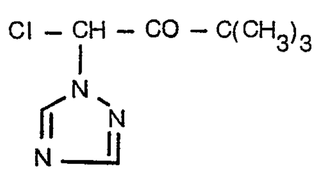

(Verfahren a)

21 g (0,125 Mol) 3,3-Dimethyl-1-(1,2,4,-triazol-1-yl)-butan-2-on und 11,3 g (0,138 Mol) Natriumacetat werden in 150 ml Eisessig gelöst und in 1,5 Stunden 9 g (0,125 Mol) Chlor eingeleitet. Die Temperatur steigt bis auf ca. 36°C an. Die weiße Suspension wird über Nacht bei 35—40°C weitergerührt, dannach eingeengt und der Rückstand mit Ethylenchlorid/Wasser/Acetatpuffer ausgeschüttelt. Die organische Phase wird abgetrennt und eingeengt, Man erhält 23 g (92% der Theorie) 1-Chlor-3,3-dimethyl-1-(1,2,4-triazol-1-yl)-butan-2-on als hellgelbes Oel, das direkt weiter umgesetzt werden kann.

Beispiel (II—5)

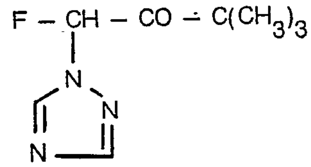

(Verfahren b)

Eine Mischung von 69 g (0,5 Mol) gepulvertem Kaliumcarbonat und 34,5 g (0,5 Mol) 1,2,4-Triazol in 250 ml Aceton wird bei 20°C unter Rühren mit 98,5 g (0,5 Mol) 1-Brom-3,3-dimethyl-1-fluor-butan-2-on versetzt. Man läßt 4 Stunden bei 20°C nachrühren. Der anorganische Niederschlag wird abfiltriert und das Filtrat eingedampft. Der Rückstand wird im Hochvakuum destilliert. Man erhält 50,3 g (54% der Theorie) 3,3-Dimethyl-1-fluor-1-(1,2,4-triazol-1-yl)-butan-2-on vom Siedepunkt 74—78°C/0,1 mm Hg-Säule und vom Schmelzpunkt 53°C.

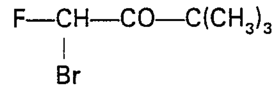

In eine Lösung von 810 g (6,86 Mol) Monofluorpinakolin in 3,5 l Ether werden bei Zimmertemperatur 1,11 kg Brom langsam eingetropft. Die Reaktion ist nur schwach exotherm. Nach der letzen Bromzugabe verschwindet die Bromfarbe rasch. Es wird noch eine Stunde nachgerührt und dann über Nacht stehen gelassen. Nach vorsichtiger Zugabe von 2000 ml Wasser wird die Etherphase abgetrennt, fünfmal mit je 150 ml Wasser gewaschen und dann über Natriumsulfat getrocknet. Nach Einengen der Etherphase verbleibt eine gelbe Flüssigkeit, die im Wasserstrahlvakuum fraktioniert destilliert wird. Man erhält 1063,5 g (78,8% der Theorie) 1-Brom-3,3-dimethyl-1-fluor-butan-2-on vom Siedepunkt 53—57°C/12 mm Hg-Säule.

**Patentansprüche**

1. Verfahren zur Herstellung von 1-Azolyl-1-phenoxy-alkan-2-onen der Formel

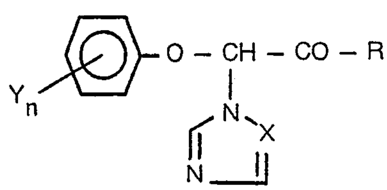

(I)

in welcher

R für Alkyl mit 1 bis 4 Kohlenstoffatomen oder für gegebenenfalls durch Halogen, Alkyl mit 1 bis 4 oder Alkoxy mit 1 bis 2 Kohlenstoffatomen substituiertes Phenyl steht,

X für ein Stickstoffatom oder die CH—Gruppe steht,

Y für Halogen, Alkyl und Alkoxy mit jeweils 1 bis 4 und Cycloalkyl mit 5 bis 7 Kohlenstoffatomen steht, für die Nitro-Gruppe und für die Gruppierung —CO—OR′ steht, ferner für Phenyl und Phenoxy steht, wobei die beiden letztgenannten Reste substituiert sein können durch Halogen oder Alkyl mit 1 bis 2 Kohlenstoffatomen,

R′ für Alkyl mit 1 bis 4 Kohlenstoffatomen und für Phenyl oder Benzyl steht, wobei die beiden letztgenannten Reste durch Halogen und Alkyl mit 1 bis 2 Kohlenstoffatomen substituiert sein können und

n für die Zahlen 0, 1, 2 oder 3 steht,

dadurch gekennzeichnet daß man Azolylhalogenketone der Formel

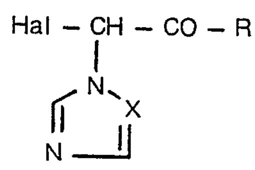

x (H–Hal)$_m$

(II)

in welcher
R und X die oben angegebene Bedeutung haben,
Hal für Fluor, Chlor oder Brom steht und
m für 0 oder 1 steht,
mit Phenolen der Formel

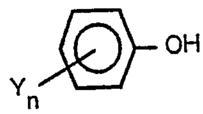

(III)

in welcher
Y und n die oben angegebene Bedeutung haben,
in Gegenwart eines inerten organischen Lösungsmittels und in Gegenwart eines anorganischen oder organischen Säurebindemittels im Temperaturbereich zwischen 0 und 140°C umsetzt.

2. Verfahren gemäß Anspruch 1, dadurch gekennzeichnet, daß man die Umsetzung im Temperaturbereich zwischen 50 und 100°C vornimmt.

3. Azolylketone der Formel

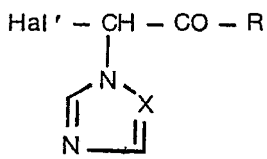

x $(H–Hal')_m$

(IIa)

in welcher
X für ein Stickstoffatom oder die CH—Gruppe steht,
R für Alkyl mit 1 bis 4 Kohlenstoffatomen oder für gegebenenfalls durch Halogen, Alkyl mit 1 bis 4 oder Alkoxy mit 1 bis 2 Kohlenstoffatomen substituiertes Phenyl steht,
Hal' für Fluor steht, sowie zusätzlich auch für Chlor und Brom steht, wenn *R* für gegebenenfalls substituiertes Phenyl steht und,
m für 0 oder 1 steht.

**Claims**

1. Process for the preparation of 1-azolyl-1-phenoxy-alkan-2-ones of the formula

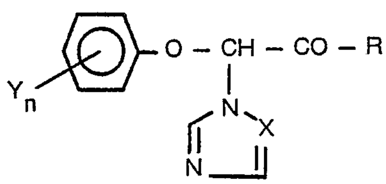

(I)

in which
R represents alkyl with 1 to 4 carbon atoms or phenyl which is optionally substituted by halogen, alkyl with 1 to 4 or alkoxy with 1 to 2 carbon atoms,
X represents a nitrogen atom or the CH group,
Y represents halogen, alkyl or alkoxy with in each case 1 to 4 or cycloalkyl with 5 to 7 carbon atoms, the nitro group or the grouping —CO—OR', or, furthermore, phenyl or phenoxy, it being possible for the two last-mentioned radicals to be substituted by halogen or alkyl with 1 to 2 carbon atoms,
R' represents alkyl with 1 to 4 carbon atoms or phenyl or benzyl, it being possible for the two last-mentioned radicals to be substituted by halogen or alkyl with 1 to 2 carbon atoms and
n represents the number 0, 1, 2 or 3,
characterised in that azolylhalogenoketones of the formula

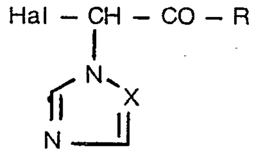

x (H–Hal)$_m$ (II)

in which

R and X have the abovementioned meaning,

Hal represents fluorine, chlorine or bromine and

m represents 0 or 1,

are reacted with phenols of the formula

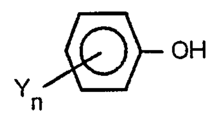

(III)

in which

Y and n have the abovementioned meaning,

in the presence of an inert organic solvent and in the presence of an inorganic or organic acid-binding agent in the temperature range of between 0 and 140°C.

2. Process according to Claim 1, characterised in that the reaction is carried out in the temperature range of between 50 and 100°C.

3. Azolyl ketones of the formula

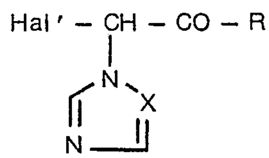

x (H–Hal')$_m$ (IIa)

in which

X represents a nitrogen atom or the CH group,

R represents alkyl with 1 to 4 carbon atoms or phenyl which is optionally substituted by halogen, alkyl with 1 to 4 or alkoxy with 1 to 2 carbon atoms,

Hal' represents fluorine, or, in addition, when *R* represents optionally substituted phenyl, chlorine or bromine, and

m represents 0 or 1.

**Revendications**

1. Procédé de préparation de 1-azolyl-1-phénoxy-alcane-2-ones de formule:

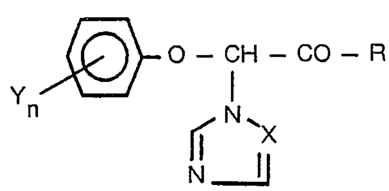

(I)

[dans laquelle

R représente un groupe alkyle ayant 1 à 4 atomes de carbone ou un groupe phényle éventuellement substitué par de l'halogène, par un groupe alkyle ayant 1 à 4 ou alcoxy ayant 1 à 2 atomes de carbone,

X représente un atome d'azote ou le groupe CH,

Y représente un halogène, un groupe alkyle et alcoxy ayant chacun 1 à 4 et cycloalkyle ayant 5 à 7 atomes de carbone, le groupe nitro et le groupement —CO—OR', ainsi que le groupe phényle et phénoxy, les deux derniers radicaux cités pouvant être substitués par de l'halogène ou par un groupe alkyle ayant 1 à 2 atomes de carbone,

R' représente un groupe alkyle ayant 1 à 4 atomes de carbone et un groupe phényle ou benzyle, les deux derniers radicaux cités pouvant être cités par de l'halogène et par un groupe alkyle ayant 1 à 2 atomes de carbone, et

n représente les nombres 0, 1, 2 ou 3],

caractérisé en ce qu'on fait réagir en présence d'un solvant organique inerte et en présence d'un agent minéral ou organique de fixation des acides, à intervalle de températures compris entre 0 et 140°C, des azolylhalogénocétones de formule:

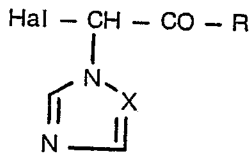

x (H–Hal)$_m$ (II)

[dans laquelle

R et X ont le sens indiqué ci-dessus,

Hal représente le fluor, le chlore ou le brome, et

m vaut 0 ou 1],

avec des phénols de formule:

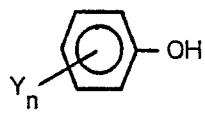

(III)

[dans laquelle

Y et n ont le sens indiqué ci-dessus].

2. Procédé selon la revendication 1, caractérisé en ce qu'on effectue la réaction dans un intervalle de températures compris entre 50 et 100°C.

3. Azolylcétones de formule:

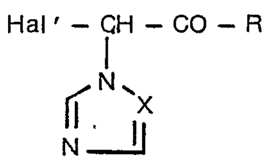

x (H–Hal')$_m$ (IIa)

[dans laquelle

X représente un atome d'azote ou le groupe CH,

R représente un groupe alkyle ayant 1 à 4 atomes de carbone ou un groupe phényle éventuellement substitué par de l'halogène, un groupe alkyle ayant 1 à 4 ou alcoxy ayant 1 à 2 atomes de carbone,

Hal' représente le fluor, et, en outre également, le chlore et le brome lorsque *R* représente un groupe phényle éventuellement substitué, et

m vaut 0 ou 1].